# EUROPEAN PATENT APPLICATION

(11) **EP 4 056 209 A1**
(43) Date of publication of application: **14.09.2022**
(21) Application number: 19952523.9
(22) Date of filing: 15.11.2019
(51) Int. Cl.: A61M 5/142

(54) **INFUSION PUMP**

(71) Applicant: Shenzhen Mindray Scientific Co., Ltd., Shenzhen, Guangdong 518000 (CN)
(72) Inventor: ZHENG, Hong, Shenzhen, Guangdong 518000 (CN); DONG, Shenhui, Shenzhen, Guangdong 518000 (CN); DENG, Nanfang, Shenzhen, Guangdong 518000 (CN); HU, Lian, Shenzhen, Guangdong 518000 (CN); LIU, Huayong, Shenzhen, Guangdong 518000 (CN); LU, Yi, Shenzhen, Guangdong 518000 (CN); ZHANG, Xuegang, Shenzhen, Guangdong 518000 (CN)
(74) Representative: KIPA AB
(86) International application number: PCT/CN2019/118999
(87) International publication number: WO 2021/092956

(57) **Abstract**

An infusion pump, comprising a pump main body (100) and a pump door (200). The pump main body (100) comprises a pump main body housing (110), a control module, an external interface assembly (120), a power supply module (130), a battery assembly (140), a lock lever assembly (150), a lock lever driving mechanism (160), and a pump body assembly (170). The side of the pump main body housing (110) facing the pump door (200) is a front cover (111). The battery assembly (140), the pump body assembly (170), and the lock lever driving mechanism (160) are arranged side-by-side behind the front cover (111). The pump body assembly (170) is disposed at the installation position of the pump main body to press an infusion tube (300). The battery assembly (140) and the lock lever driving mechanism (160) are respectively disposed on two sides of the pump body assembly (170). The lock lever assembly (150) is located above the pump body assembly (170) and the lock lever driving mechanism (160). The power supply module (130) and the external interface assembly (120) are arranged side-by-side, one of which is located behind the pump body assembly (170), and the other is located behind the lock lever driving mechanism (160). The overall layout is set according to the volume and shape of each component, and the advantages and disadvantages of the volume and shape of each component can be fully used, making the overall structure more compact and the whole machine smaller.

## Description

### TECHNICAL FIELD

The disclosure relates to the field of medical devices, and in particular to a structure of an infusion pump.

### BACKGROUND

Infusion pumps are a type of widely applied fluid infusion devices. Typically, an infusion pump includes a pump main body and a pump door. The pump main body generally has an infusion tube installation structure and other required components including, for example, a pump body assembly, a driving mechanism configured to control the pump door to be locked to the pump main body, a power supply module, a battery assembly, etc.

Due to a great variety of medical devices at present and the limited placement space of relevant places in hospitals, there is an increasing demand for miniaturization and compactness of various infusion pumps at present. In the existing infusion pumps, the structural layout thereof needs to be further optimized and improved.

### SUMMARY

### TECHNICAL PROBLEM

The disclosure mainly provides a new-type infusion pump for improving the compactness of structure of the infusion pump.

### SOLUTION TO THE PROBLEM

### TECHNICAL SOLUTION

An embodiment of the disclosure provides an infusion pump, including a pump main body and a pump door mounted on the pump main body in a lockable and openable manner, where
the pump main body includes a pump main body housing, a control module, an external interface assembly, a power supply module, a battery assembly, a lock lever assembly, a lock lever driving mechanism, and a pump body assembly; the pump main body housing has an installation cavity, the side of the pump main body housing facing the pump door is a front cover, and the front cover has an infusion tube installation structure for installation of an infusion tube and a pump main body installation position for installation of the pump body assembly; the control module, the external interface assembly, the power supply module, the battery assembly, the lock lever assembly, the lock lever driving mechanism and the pump body assembly are all installed in the installation cavity; the battery assembly, the pump body assembly and the lock lever driving mechanism are arranged side-by-side behind the front cover, and the pump body assembly is disposed at the pump main body installation position to press the infusion tube; the battery assembly and the lock lever driving mechanism are respectively disposed on two sides of the pump body assembly; the lock lever assembly is located above the pump body assembly and the lock lever driving mechanism, and the lock lever driving mechanism drives the lock lever assembly to move so as to lock the lock lever assembly to or unlock same from the pump door; the power supply module and the external interface assembly are arranged side-by-side, one of which is located behind the pump body assembly, and the other is located behind the lock lever driving mechanism; and
the pump door has a door body, a lock catch assembly and a tube pressing assembly, the lock catch assembly and the tube pressing assembly are installed on the door body, the lock catch assembly is configured to be locked to the lock lever assembly, and the tube pressing assembly is disposed corresponding to the position of the pump body assembly and is configured to press the infusion tube together with the pump body assembly.

In an embodiment, the pump main body further includes a fluid stop assembly, where the front cover is divided into an upstream region and a downstream region, with the pump body assembly as a boundary, according to the flow direction of fluid in the infusion tube, and the fluid stop assembly is located in the downstream region of the front cover and exposed from the front cover so as to stop the flow of fluid in the infusion tube.

In an embodiment, the fluid stop assembly is installed in a gap between the lock lever driving mechanism and the front cover.

In an embodiment, the pump main body further includes an upper pressure measurement assembly and a lower pressure measurement assembly, where the upper pressure measurement assembly is located in the upstream region of the front cover, and the lower pressure measurement assembly is located in the downstream region of the front cover and located upstream of the fluid stop assembly, and the upper pressure measurement assembly and the lower pressure measurement assembly are exposed from the front cover so as to measure the pressure of fluid in the infusion tube; and the door body is provided with an upper pressure measurement press block corresponding to the upper pressure measurement assembly and a lower pressure measurement press block corresponding to the lower pressure measurement assembly.

In an embodiment, the upper pressure measurement assembly is installed in a gap between the battery assembly and the front cover.

In an embodiment, the lower pressure measurement assembly is installed in the gap between the lock lever driving mechanism and the front cover.

In an embodiment, the pump main body further includes an upper ultrasound detection assembly and a lower ultrasound detection assembly, where the upper ultrasound detection assembly is located in the upstream region of the front cover and located downstream of the upper pressure measurement assembly; the lower ultrasound detection assembly is located in the downstream region of the front cover and located upstream of the lower pressure measurement assembly; the upper ultrasound detection assembly and the lower ultrasound detection assembly are exposed from the front cover so as to detect air bubbles in the infusion tube; and the door body is provided with an upper ultrasound detection press block corresponding to the upper ultrasound detection assembly and a lower ultrasound detection press block corresponding to the lower ultrasound detection assembly.

In an embodiment, the pump main body further includes an upper ultrasound detection assembly and a lower ultrasound detection assembly, where the upper ultrasound detection assembly is located in the upstream region of the front cover and located in upstream of the upper pressure measurement assembly; the lower ultrasound detection assembly is located in the downstream region of the front cover and located between the lower pressure measurement assembly and the fluid stop assembly; the upper ultrasound detection assembly and the lower ultrasound detection assembly are exposed from the front cover so as to detect air bubbles in the infusion tube; and the door body is provided with an upper ultrasound detection press block corresponding to the upper ultrasound detection assembly and a lower ultrasound detection press block corresponding to the lower ultrasound detection assembly.

In an embodiment, the upper ultrasound detection assembly is installed in the gap between the battery assembly and the front cover.

In an embodiment, the lower ultrasound detection assembly is installed in the gap between the lock lever driving mechanism and the front cover.

In an embodiment, the pump main body further includes an upper tube in-position detection assembly and a lower tube in-position detection assembly, where the upper tube in-position detection assembly is located in the upstream region of the front cover and located upstream of the upper pressure measurement assembly and the upper ultrasound detection assembly; the lower tube in-position detection assembly is located in the downstream region of the front cover and located downstream of the fluid stop assembly; and the upper tube in-position detection assembly and the lower tube in-position detection assembly are exposed from the front cover so as to detect whether the infusion tube is installed in position.

In an embodiment, the upper tube in-position detection assembly is installed in the gap between the battery assembly and the front cover.

In an embodiment, the lower tube in-position detection assembly is installed on the rear side of the front cover and located on the outer side of the lock lever driving mechanism.

In an embodiment, the upper tube in-position detection assembly and the lower tube in-position detection assembly are respectively disposed at the outermost ends of two sides of the front cover.

In an embodiment, the pump main body further includes a drop counting sensor disposed behind the upper tube in-position detection assembly.

In an embodiment, at least part of the pump main body housing is a first metal installation base made of a metal material, and the pump body assembly, the lock lever assembly and the lock lever driving mechanism are installed on the first metal installation base; at least part of the door body of the pump door is a second metal installation base made of a metal material, and the pressure tube and the lock catch assembly are installed on the second metal installation base;

One end of the first metal installation base is rotatably connected to the second metal installation base to enable the door body to rotate relative to the pump main body; there are a locking position and an unlocking position on a movement path of the second metal installation base; in the locking position, the lock lever assembly is locked to the lock catch assembly, the tube pressing assembly and the pump body assembly are located on two sides of the infusion tube, and when the pump body assembly is started, the infusion tube is pressed so as to perform infusion; and in the unlocking position, the lock lever assembly is unlocked from the lock catch assembly such that the tube pressing assembly can move away from one side of the infusion tube to release the infusion.

In an embodiment, the pump main body further includes a first metal installation base, which is installed in the pump main body housing, and on which the pump body assembly, the lock lever assembly, and the lock lever driving mechanism are installed; the pump door includes a second metal installation base, which is disposed in the door body, and on which the tube pressing assembly and the lock catch assembly are installed;
one end of the first metal installation base is rotatably connected to the second metal installation base to enable the door body to rotate relative to the pump main body; there are a locking position and an unlocking position on a movement path of the second metal installation base; in the locking position, the lock lever assembly is locked to the lock catch assembly, the tube pressing assembly and the pump body assembly are located on two sides of the infusion tube, and when the pump body assembly is started, the infusion tube is pressed so as to perform infusion; and in the unlocking position, the lock lever assembly is unlocked from the lock catch assembly such that the tube pressing assembly can move away from one side of the infusion tube to release the infusion tube.

In an embodiment, the first metal installation base includes an upper metal member and a lower metal member, the pump body assembly is fixedly installed on the lower metal member or the upper metal member, the lock lever assembly is movably installed on the upper metal member, the upper metal member is fixedly installed on the lower metal member, and the second metal installation base is hinged to the lower metal member.

In an embodiment, the lower metal member is substantially of a U-shaped structure, the upper metal member is substantially of an inverted U-shaped structure, and the upper metal member and the lower metal member are matched with each other and fixedly connected by means of the metal members.

In an embodiment, the lock lever driving mechanism has a driving member and an output member connected to the driving member in a transmission manner, the lock lever assembly is rotatably installed on the output member, and the output member drives the lock lever assembly to move in a straight reciprocating motion along a first axis in a horizontal plane to enable the lock lever assembly to be locked to the lock catch or unlocked from the lock catch; the first axis passes through an axial center of the output member; and the lock lever assembly is located, relative to a center line of rotation of the output member, in the same horizontal plane as the first axis and perpendicular to the first axis.

In an embodiment, the output member and the lock lever assembly are in line contact and/or point contact therebetween, contact positions of the line contact and/or contact positions of the point contact are symmetrically distributed on two sides of the first axis and located in the same horizontal plane as the first axis, and a connecting line of the contact positions between the output member and the lock lever assembly is perpendicular to the first axis.

In an embodiment, the lock lever driving mechanism includes a lead screw and nut transmission mechanism, where an electric motor serves as the driving member, a nut in the lead screw and nut transmission mechanism serves as the output member, the lock lever assembly is installed on the nut, the electric motor drives a lead screw in the lead screw and nut transmission mechanism so as to drive the nut and the lock lever assembly to move, and the first axis coincides with a central axis of the lead screw in the lead screw and nut transmission mechanism.

In an embodiment, two sides of the nut are respectively provided with a protrusion, the lock lever assembly is provided with bayonets corresponding to the protrusions, the bayonet is sleeved on an outer wall of the protrusion, and an inner wall of the bayonet is in line contact with the outer wall of the protrusion.

In an embodiment, the bayonet has a U-shaped opening fastened and sleeved on the protrusion.

In an embodiment, the lock lever assembly has a snap-fit fastener, which has a snap-fit surface configured to be in contact with the corresponding lock catch assembly and fasten the snap-fit surface of the lock catch assembly, the snap-fit surface has a first inclined surface and a second inclined surface, the first inclined surface is disposed at an outer end of the snap-fit surface, the second inclined surface is connected to an inward end of the first inclined surface, and the angle of inclination of the first inclined surface is greater than that of the second inclined surface.

In an embodiment, the lock lever assembly includes a lock lever and a connecting member, where the lock lever has the snap-fit fastener, the lock lever is connected to the connecting member, and the connecting member is rotatably connected to the output member to enable the output member to drive the connecting member and the lock lever to move.

In an embodiment, the pump body assembly includes a pump installation base, a peristaltic pump and an elastic member, where the pump installation base is fixedly installed on the first metal installation base, the peristaltic pump is installed on the pump installation base, and the elastic member acts between the peristaltic pump and the first metal installation base to apply an elastic restoring force to the peristaltic pump for driving the peristaltic pump to move towards the tube pressing assembly.

### BENEFICIAL EFFECTS OF THE INVENTION

### BENEFICIAL EFFECTS

The infusion pump according to the above embodiments includes a pump main body and a pump door. The pump main body includes a pump main body housing, a control module, an external interface assembly, a power supply module, a battery assembly, a lock lever assembly, a lock lever driving mechanism, and a pump body assembly. The side of the pump main body housing facing the pump door is a front cover, the battery assembly, the pump body assembly and the lock lever driving mechanism are arranged side-by-side behind the front cover, and the pump body assembly is disposed at a pump main body installation position to press an infusion tube. The battery assembly and the lock lever driving mechanism are respectively disposed on two sides of the pump body assembly. The lock lever assembly is located above the pump body assembly and the lock lever driving mechanism. The power supply module and the external interface assembly are arranged side-by-side, one of which is located behind the pump body assembly, and the other is located behind the lock lever driving mechanism. The overall layout is set according to the volume and shape of each component, the advantages and disadvantages of the volume and shape of each component can be made fully used, making the overall structure more compact and the whole machine smaller.

### BRIEF DESCRIPTION OF THE DRAWINGS

FIGS. 1 and 2 are schematic structural diagrams of an infusion pump (with a pump door in an open state) in an embodiment of the disclosure;
FIGS. 3 and 4 are schematic diagrams of an internal structural layout of a pump body in an embodiment of the disclosure;
FIG. 5 is a schematic structural diagram of fitting of a first metal installation base and a second metal installation base (in an unlocked state) in an embodiment of the disclosure;
FIG. 6 is a schematic structural diagram of fitting of a first metal installation base and a second metal installation base (in a locked state) in an embodiment of the disclosure;
FIG. 7 is a schematic diagram of installation of a pump body assembly in an embodiment of the disclosure;
FIG. 8 is a schematic structural diagram of fitting of a lock lever assembly and a lock catch assembly in a locked state in an embodiment of the disclosure;
FIG. 9 is a schematic structural diagram of installation of a pump body assembly in an embodiment of the disclosure;
FIG. 10 is a schematic structural diagram of a pump body assembly in an embodiment of the disclosure;
FIG. 11 is a schematic structural diagram of fitting of a lock lever assembly and a lock lever driving mechanism in an embodiment of the disclosure;
FIG. 12 is a schematic structural diagram of assembly of a lock lever assembly and a lead screw and nut transmission mechanism in an embodiment of the disclosure;
FIG. 13 is a partial cross-sectional view of a lead screw in a lead screw and nut transmission mechanism in an embodiment of the disclosure; and
FIG. 14 is a schematic structural diagram of a lock lever snap-fit surface in an embodiment of the disclosure.

### EMBODIMENTS OF THE INVENTION

### DESCRIPTION OF THE PREFERRED EMBODIMENTS

The disclosure will be further described in detail below through specific implementations in conjunction with the accompanying drawings. Associated similar element reference numerals are used for similar elements in different implementations. In the following implementations, many details are described such that the disclosure may be better understood. However, it may be effortlessly appreciated by persons skilled in the art that some of the features may be omitted, or may be substituted by other elements, materials, and methods in different cases. In certain cases, some operations involved in the disclosure are not displayed or described in the specification, which is to prevent a core part of the disclosure from being obscured by too much description. Moreover, for persons skilled in the art, the detailed description of the involved operations is not necessary, and the involved operations can be thoroughly understood according to the description in the specification and general technical knowledge in the art.

In addition, the characteristics, operations, or features described in the specification may be combined in any appropriate manner to form various implementations. Meanwhile, the steps or actions in the method description may also be exchanged or adjusted in order in a way that is obvious to persons skilled in the art. Therefore, the various orders in the specification and the accompanying drawings are merely for the purpose of clear description of a certain embodiment and are not meant to be a necessary order unless it is otherwise stated that a certain order must be followed.

The serial numbers themselves for the components herein, for example, "first" and "second", are merely used to distinguish the described objects, and do not have any sequential or technical meaning. Moreover, as used in the disclosure, "connection" or "coupling", unless otherwise stated, includes both direct and indirect connections (couplings).

This embodiment provides an infusion pump. The infusion pump is configured to control parameters, such as the number of infusion drop count or infusion flow rate, so as to ensure that the drug can enter a patient's body accurately and safely according to set speed and dose.

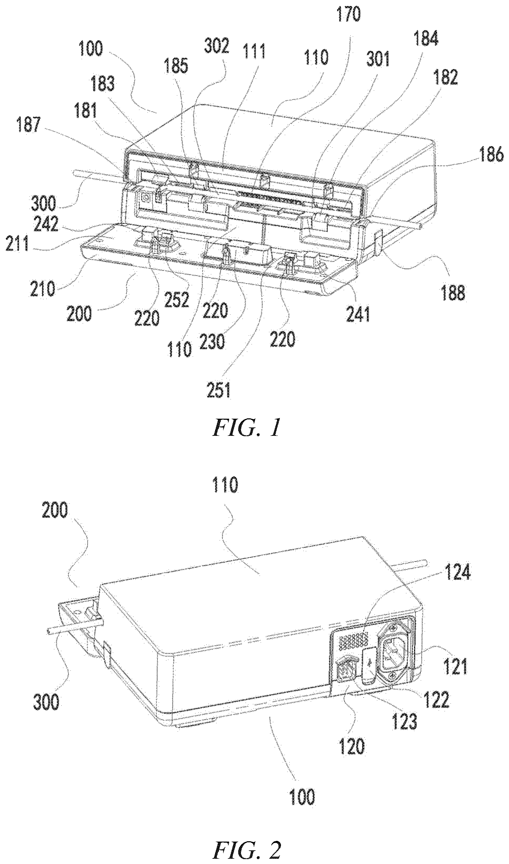

Referring to FIGS. 1 to 4, the infusion pump includes a pump main body 100 and a pump door 200. The pump door 200 is installed on the pump main body 100 in a lockable and openable manner. In this lockable and openable manner, the pump door 200 is generally rotatably installed on the pump main body 100 such that the pump door 200 can rotate to open and close the pump door 200. When infusion is required, the pump door 200 is moved to a corresponding locking position and locked to the pump main body 100 to perform the infusion. Of course, in some embodiments, the lockable and openable manner is not limited to the case where the pump door 200 is rotatably connected to the pump main body 100, and the pump door 200 may be locked to and unlocked from the pump main body 100 in a sliding, flipped or folded manner. Furthermore, in other embodiments, in the lockable and openable manner, the pump door 200 may be connected to the pump main body 100 in a removable manner, that is, the pump door 200 may be removed from the pump main body 100 in an opened state, and the pump door 200 can be installed on the pump main body 100 when it needs to be locked.

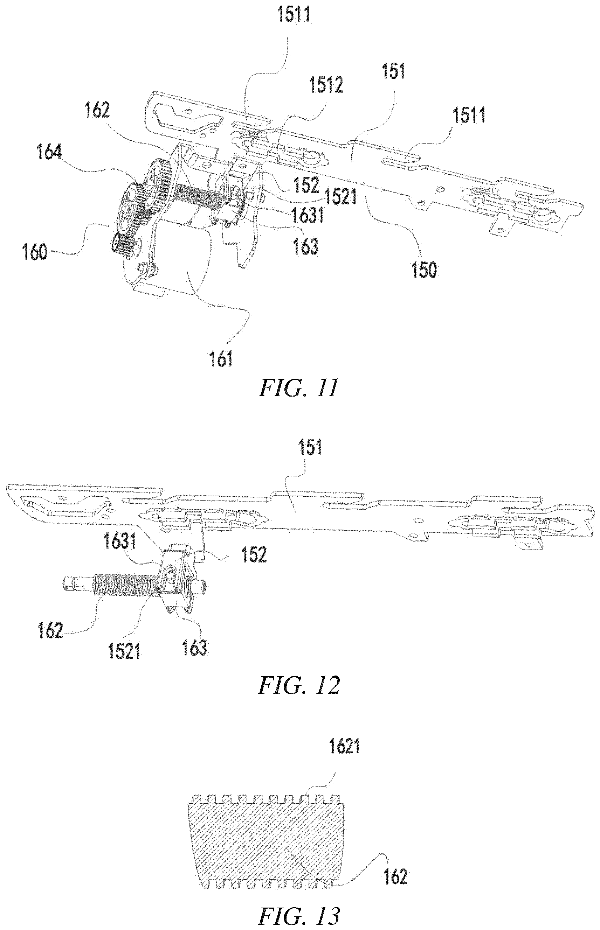

With continued reference to FIGS. 1 to 6, the pump main body 100 includes a pump main body housing 110, a control module (not shown in figures), an external interface assembly 120, a power supply module 130, a battery assembly 140, a lock lever assembly 150 (in conjunction with FIG. 11), a lock lever driving mechanism 160, and a pump body assembly 170.

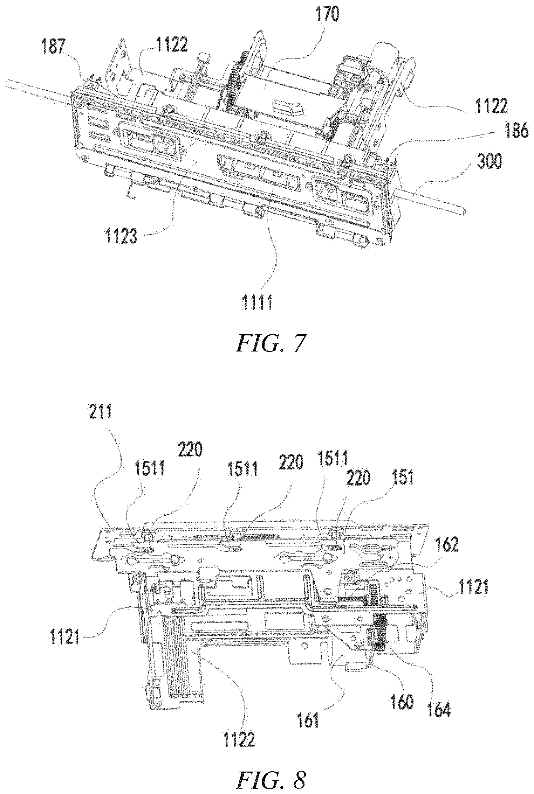

The pump main body housing 110 has an installation cavity for installation of components. The side of the pump main body housing 110 facing the pump door 200 is a front cover 111, and the front cover 111 has an infusion tube installation structure for installation of an infusion tube and a pump main body installation position for installation of the pump body assembly 170. The infusion tube installation structure is configured to stabilize the infusion tube on the pump main body 100 for infusion and various detections. As shown in FIG. 1, the infusion tube 300 is placed on the infusion tube installation structure of the front cover 111. Generally, the infusion tube installation structure may be a holding structure or another installation structures, which are not described in detail herein. The pump main body installation position is typically an installation window 1111 (as shown in FIG. 7), and the portion of the pump main body 100 that is configured to press the infusion tube 300 may extend from the installation window 1111 so as to press the infusion tube 300.

The control module is the entire control center of the infusion pump, which typically has a processor, an associated control circuit, etc. The control module is connected to other modules, assemblies, or mechanisms that need to be controlled, so as to control the modules, assemblies, or mechanisms according to system settings. The external interface assembly 120 is mainly configured to be in signal communication with an external device and to supply power to the infusion pump. The external interface assembly 120 may provide different interfaces as desired. For example, in some embodiments, referring to FIG. 2, the external interface assembly 120 may include a power supply interface 121, a USB interface 122, a multi-functional interface 123, etc. In addition, in order to further save space and make the infusion pump more compact, in an embodiment, the external interface assembly 120 is further provided with a loudspeaker 124 for playing sound signals without additionally providing a loudspeaker at another position of the infusion pump.

The power supply module 130 is connected to the control module for supplying power to a system and other electrical components. The battery assembly 140 has a function of power storage, typically supplying power when the infusion pump is powered off. Of course, in a non-powered-off state, the battery assembly 140 may also be used for power supply. The lock lever assembly 150 is configured to be locked to the pump door 200 during operation, and the lock lever driving mechanism 160 is configured to drive the lock lever assembly 150 to move so as to switch the lock lever assembly 150 between a locking position and an unlocking position. The pump body assembly 170 is configured to apply a pressing force to the infusion tube 300 so as to drive the fluid in the infusion tube 300 to flow forwards. The pump body assembly 170 may use a variety of structures that can press an infusion pump, such as a peristaltic pump used frequently.

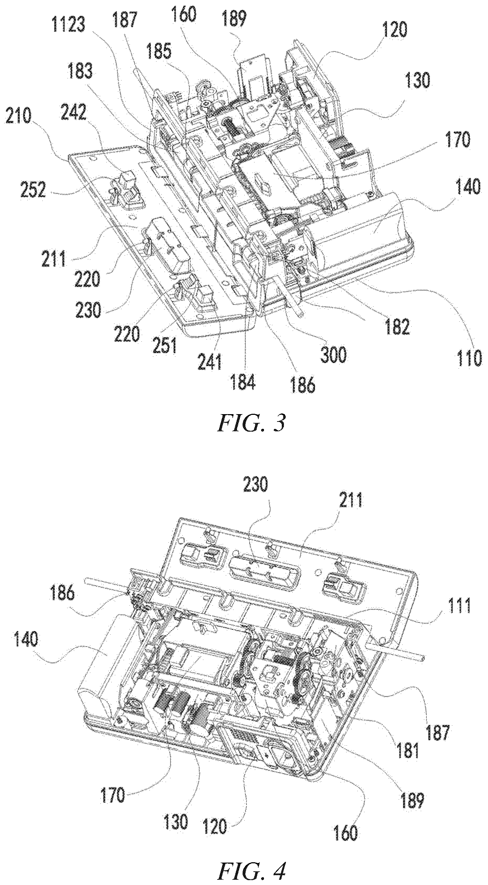

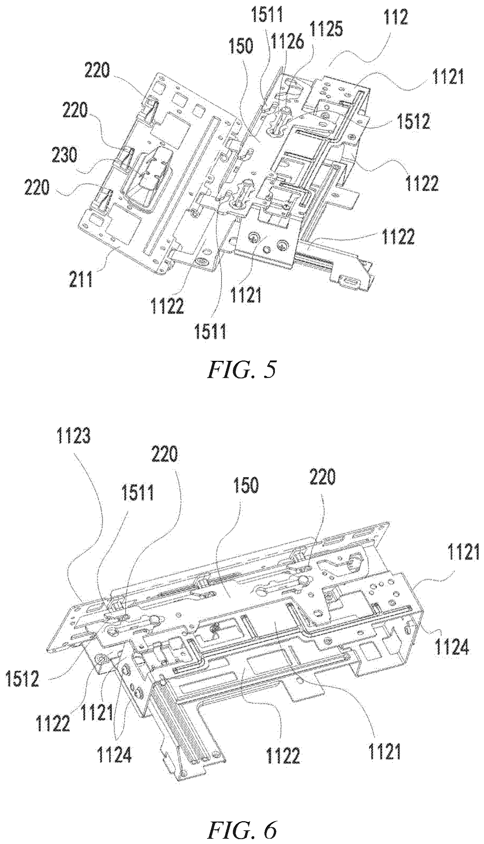

The control module, the external interface assembly 120, the power supply module 130, the battery assembly 140, the lock lever assembly 150, the lock lever driving mechanism 160, and the pump body assembly 170 are all installed in the installation cavity. The battery assembly 140, the pump body assembly 170, and the lock lever driving mechanism 160 are arranged side-by-side behind the front cover 111. The pump body assembly 170 is disposed at the pump main body installation position to press the infusion tube 300. The battery assembly 140 and the lock lever driving mechanism 160 are respectively disposed on two sides of the pump body assembly 170. The lock lever assembly 150 is located above the pump body assembly 170 and the lock lever driving mechanism 160 (as shown in conjunction with FIGS. 5 and 6), and the lock lever driving mechanism 160 drives the lock lever assembly 150 to move so as to lock the lock lever assembly 150 to or unlock same from the pump door 200. The power supply module 130 and the external interface assembly 120 are arranged side-by-side, one of which is located behind the pump body assembly 170, and the other is located behind the lock lever driving mechanism 160. In FIGS. 3 and 4, the external interface assembly 120 is located behind the lock lever driving mechanism 160, and the power supply module 130 is located behind the pump body assembly 170. In other embodiments, the positions of the external interface assembly and the power supply module may be interchanged.

Accordingly, referring to FIGS. 1 and 2, the pump door 200 has a door body 210, a lock catch assembly 220, and a tube pressing assembly 230. The lock catch assembly 220 and the tube pressing assembly 230 are installed on the door body 210. The door body 210 may be connected, rotatably or in other manners, to the pump main body housing 110. The lock catch assembly 220 is configured to be locked to the lock lever assembly 150, and the tube pressing assembly 230 is disposed corresponding to the position of the pump body assembly 170 and configured to press the infusion tube 300 together with the pump body assembly 170. The tube pressing assembly 230 may be a tube pressing block or an infusion tube pressing apparatus of other structures.

In the embodiment described above, the overall layout is set according to the volume and shape of each component, the advantages and disadvantages of the volume and shape of each component can be made fully used, making the overall structure more compact and the whole machine smaller.

Further, referring to FIGS. 1 and 4, in an embodiment, the pump main body 100 further includes a fluid stop assembly 181. For the convenience of description, in the disclosure, according to the flow direction of fluid in the infusion tube 300, the front cover 111 is divided into an upstream region and a downstream region, with the pump body assembly 170 as a boundary, that is, the portion of the infusion tube 300 where the fluid flows to the pump body assembly 170 is the upstream 301, the portion thereof where the flow flows away from the pump body assembly 170 is the downstream 302, the region of the front cover 111 corresponding to the upstream 301 of the infusion tube 300 is the upstream region, and the region of the front cover 111 corresponding to the downstream 302 of the infusion tube 300 is the downstream region. From the view shown in FIG. 1, the fluid in the infusion tube 300 flows from right to left, and the portion of the front cover 111 that is located on the right side of the pump body assembly 170 is the upstream region, and the portion thereof located on the left side of the pump body assembly 170 is the downstream region. The fluid stop assembly 181 is located in the downstream region of the front cover 111 and exposed from the front cover 111 to stop the flow of fluid in the infusion tube 300. In terms of the flow direction of the fluid in the infusion tube 300, the fluid stop assembly 181 being disposed downstream of the pump body assembly 170 facilitates stopping the flow of more fluid. When no infusion is needed, the flow of fluid in the tube may be stopped quickly to prevent excess fluid from flowing into the patient. In particular, the fluid stop assembly 181 is disposed on the outer side of the downstream region as much as possible to minimize the flow of fluid to the patient when no infusion is needed, ensuring the product safety.

Referring to FIG. 4, in an embodiment, the fluid stop assembly 181 is installed in a gap between the lock lever driving mechanism 160 and the front cover 111. The fluid stop assembly 181 is located in the right front or directly in front of the lock lever driving mechanism 160 (with the side where the front cover 111 is located being the front, and the side opposite to the front cover 111 being the rear, the same below), such that the space between the lock lever driving mechanism 160 and the front cover 111 can be fully utilized.

In the embodiment described above, the fluid stop assembly 181 may use a fluid stop clip or other forms of fluid stop structures. Furthermore, in some embodiments, the infusion pump may not be provided with the fluid stop assembly 181, but is manually and additionally provided with a fluid stop structure to the portion of the infusion tube 300 that is located on the outer side the infusion pump to stop the flow of fluid in the infusion tube 300.

Further, in order to improve the safety of the infusion pump and to monitor the infusion state, referring to FIGS. 1 to 4, in an embodiment, the pump main body 100 further includes an upper pressure measurement assembly 182 and a lower pressure measurement assembly 183. The upper pressure measurement assembly 182 is located in the upstream region of the front cover 111. The lower pressure measurement assembly 183 is located in the downstream region of the front cover 111 and located upstream of the fluid stop assembly 181. The upper pressure measurement assembly 182 and the lower pressure measurement assembly 183 are exposed from the front cover 111 so as to detect the pressure of the fluid in the infusion tube 300. The door body 210 is provided with an upper pressure measurement press block 241 corresponding to the upper pressure measurement assembly 182 and a lower pressure measurement press block 242 corresponding to the lower pressure measurement assembly 183.

The upper pressure measurement assembly 182 and the lower pressure measurement assembly 183 may use a pressure sensor or other components to detect the pressure in the infusion tube 300. The upper pressure measurement assembly 182 mainly detects pressure data before the infusion tube 300 is pressurized by the pump body assembly 170, while the lower pressure measurement assembly 183 mainly detects pressure data after the infusion tube 300 is pressurized by the pump body assembly 170. Accurate pressure data may be obtained by means of combination analysis on these data, so as to achieve better adjustment for infusion parameters of the infusion pump and to improve the safety in use for the patient.

Referring to FIGS. 3 and 4, in an embodiment, the upper pressure measurement assembly 182 is installed in the gap between the battery assembly 140 and the front cover 111. In an embodiment, the lower pressure measurement assembly 183 is installed in the gap between the lock lever driving mechanism 160 and the front cover 111. The compactness of the whole machine is further improved.

Further, in order to improve the safety of the infusion pump and to monitor the infusion state, referring to FIGS. 1 to 4, in an embodiment, the pump main body 100 further includes an upper ultrasound detection assembly 184 and a lower ultrasound detection assembly 185. The upper ultrasound detection assembly 184 is located in the upstream region of the front cover 111 and located downstream of the upper pressure measurement assembly 182. The lower ultrasound detection assembly 185 is located in the downstream region of the front cover 111 and located upstream of the lower pressure measurement assembly 183. The upper ultrasound detection assembly 184 and the lower ultrasound detection assembly 185 are exposed from the front cover 111 so as to detect air bubbles in the infusion tube 300. The door body 210 is provided with an upper ultrasound detection press block 251 corresponding to the upper ultrasound detection assembly 184 and a lower ultrasound detection press block 252 corresponding to the lower ultrasound detection assembly 185.

Such a pressure and ultrasound dual-detection method can not only measure the pressure in the tube, but also detect the air bubbles in the tube, which can improve the accuracy of air bubble detection and pressure measurement and make the product safer.

In the embodiment described above, the upper ultrasound detection assembly 184 and the lower ultrasound detection assembly 185 are respectively located on the outer sides of the upper pressure measurement assembly 182 and the lower pressure measurement assembly 183, and in practice, the positions of the ultrasound detection assemblies and the pressure measurement assemblies may be interchanged. For example, in an embodiment, the upper ultrasound detection assembly 184 is located in the upstream region of the front cover 111 and located upstream of the upper pressure measurement assembly 182. The lower ultrasound detection assembly 185 is located in the downstream region of the front cover 111 and located between the lower pressure measurement assembly 183 and the fluid stop assembly 181.

Referring to FIGS. 3 and 4, in an embodiment, the upper ultrasound detection assembly 184 is installed in the gap between the battery assembly 140 and the front cover 111. In an embodiment, the lower ultrasound detection assembly 185 is installed in the gap between the lock lever driving mechanism 160 and the front cover 111.

Further, referring to FIGS. 1 to 4, in an embodiment, the pump main body 100 further includes an upper tube in-position detection assembly 186 and a lower tube in-position detection assembly 187. The upper tube in-position detection assembly 186 is located in the upstream region of the front cover 111 and located upstream of the upper pressure measurement assembly 182 and the upper ultrasound detection assembly 184. The lower tube in-position detection assembly 187 is located in the downstream region of the front cover 111 and located downstream of the fluid stop assembly 181. The upper tube in-position detection assembly 186 and the lower tube in-position detection assembly 187 are exposed from the front cover 111 to detect whether the infusion tube 300 is installed in position.

Referring to FIGS. 3 and 4, in an embodiment, the upper tube in-position detection assembly 186 is installed in the gap between the battery assembly 140 and the front cover 111. In an embodiment, the lower tube in-position detection assembly 187 is installed on the rear side of the front cover 111 and located on the outer side of the lock lever driving mechanism 160.

The upper tube in-position detection assembly 186 and the lower tube in-position detection assembly 187 may detect and indicate whether the infusion tube 300 is installed at a correct position or not. However, the positions where the infusion tube 300 is most difficult to install are two outermost sides of the machine. Therefore, as shown in FIG. 1, in an embodiment, the upper tube in-position detection assembly 186 and the lower tube in-position detection assembly 187 are respectively disposed at the outermost ends of two sides of the front cover 111 so as to effectively realize in-position monitoring of the tube and to improve the product safety.

Further, referring to FIGS. 1 to 4, in an embodiment, the pump main body 100 further includes a drop counting sensor 188 disposed behind the upper tube in-position detection assembly 186. It can be configured to check a fluid dripping state and to count fluid drops.

Further, referring to FIGS. 3 and 4, in an embodiment, the pump main body 100 further includes a wireless signal module 189, such as a WIFI module and Bluetooth module, which is disposed on the outer side of the lock lever driving mechanism 160 and in front of the external interface assembly 120 (which may alternatively be the power supply module 130), fully utilizing this space.

In addition, the pump main body housing 110 is typically located on the outer side of the pump main body 100, and the control module, the external interface assembly 120, the power supply module 130, the battery assembly 140, the lock lever assembly 150, the lock lever driving mechanism 160, and the pump body assembly 170 are all directly or indirectly installed on the pump main body housing 110. The pump door 200 is also typically installed on the pump main body housing 110 for relative movement.

The fluid flow in the infusion pump is driven by means of the pressing force applied to the infusion tube 300 by the pump body assembly 170 (such as a peristaltic pump) and the tube pressing block used in combination with the pump body assembly 170, where the pump body assembly 170 and the tube pressing block respectively press the infusion tube 300 from the two sides of the infusion tube 300, that is, the infusion tube 300 will generate a corresponding counter-acting force on the pump body assembly 170 and the tube pressing block respectively. Generally, the pump main body housing 110 is made of plastic, and the pump body assembly 170 is installed on the plastic pump main body housing 110 by means of a threaded member; when the pump body assembly 170 receives a counter-acting force from the infusion tube 300 for a long time during operation, this force is equivalent to an alternating load; when the threaded member for fixing the pump body assembly 170 on the plastic pump main body housing 110 is impacted by the alternating load for a long time, the pump main body housing 110 is prone to cracking. Thus, it is possible to cause displacement of the pump main body housing 110. When the pump door 200 and the pump body are locked, the distance between the pump main body housing 110 and the tube pressing block becomes larger, especially when the distance between the pump main body housing 110 and the tube pressing block is increased to cause that the flow in the infusion tube 300 cannot be stopped, the fluid in the infusion tube 300 will generate a free flow, which causes great harm to the patient.

Therefore, referring to FIGS. 5 to 8, in an embodiment, the pump main body 100 further includes a first metal installation base 112. The first metal installation base 112 is installed in the pump main body housing 110. At least the pump body assembly 170, the lock lever assembly 150, and the lock lever driving mechanism 160 are installed on the first metal installation base 112. The pump door 200 includes a second metal installation base 211, where the second metal installation base 211 is disposed in the door body 210, and at least the tube pressing assembly 230 and the lock catch assembly 220 are installed on the second metal installation base 211. In this embodiment, the pump main body housing 110 and the door body 210 are not limited to a metal material, but may still be made of plastic.

Furthermore, in another embodiment, at least part of the pump main body housing 110 is the first metal installation base made of the metal material. That is, the pump main body housing 110 may be partially or entirely made of the metal material to form a first metal installation base. For example, the first metal installation base may serve as one or more of the front cover 111, a bottom cover, a top cover, and a rear cover of the pump main body 100. At least the pump body assembly 170, the lock lever assembly 150, and the lock lever driving mechanism 160 are installed on the first metal installation base. Other components may also be installed on the first metal installation base or other portions (for example, non-metal portions present) of the pump main body housing 110. Similarly, at least part of the door body 210 of the pump door 200 is a second metal installation base made of the metal material, that is, the door body 210 may be partially or entirely made of the metal material to form the second metal installation base. The tube pressing assembly 230 and the lock catch assembly 220 are installed on the second metal installation base.

Further, one end of the first metal installation base 112 is rotatably connected to the second metal installation base 211 such that the door body 210 rotates relative to the pump main body 100. There are a locking position and an unlocking position on a movement path of the second metal installation base 211; in the locking position, the lock lever assembly 150 is locked to the lock catch assembly 220, the tube pressing assembly 230 and the pump body assembly 170 are located on two sides of the infusion tube 300, and when the pump body assembly 170 is started, the infusion tube 300 is pressed so as to perform infusion; and in the unlocking position, the lock lever assembly 150 is unlocked from the lock catch assembly 220 such that the tube pressing assembly 230 can move away from one side of the infusion tube 300 to release the infusion.

Since metal parts have good strength and rigidity and are not prone to deformation, cracking and breakage after receiving a force, it is possible to ensure that the pump body assembly 170 and the tube pressing assembly 230, when receive a counter-acting force of the infusion tube 300, still reliably fixed to the corresponding first metal installation base 112 and second metal installation base 211. Moreover, the counter-acting force of the infusion tube 300 against the pump body assembly 170 and the tube pressing assembly 230 will be respectively transferred to the first metal installation base 112 and the second metal installation base 211, and at this time point, the first metal installation base 112 and the second metal installation base 211 are connected with each other so as to form a closed-loop transfer of the force in cooperation with the high strength and rigidity characteristics of the metal material, which partially counteracts the acting force so as to reduce damage of metal brackets and components under force and ensure the fixing reliability of the pump body assembly 170, thereby ensuring the product safety.

Further, referring to FIG. 5, in an embodiment, the lock lever assembly 150 is movably installed on the pump main body housing 110 or the first metal installation base 112. The pump main body housing 110 or the first metal installation base 112 has a guide limiting portion 1125 disposed in a protruding manner, the lock lever assembly 150 has a corresponding guide opening 1512, the guide opening 1512 is sleeved on the guide limiting portion 1125, and a wear-resistant member 1126 is installed in the region in the guide opening 1512 which is in contact with the guide limiting portion 1125.

Further, the first metal installation base 112 and the second metal installation base 211 may be integrally formed structures or may also be formed of multiple metal assemblies connected fixedly. The first metal installation base 112 and the second metal installation base 211 may use sheet metal members, die-pressed castings, machined parts, profiles, etc.

Referring to FIGS. 5 to 8, in an embodiment, the first metal installation base 112 includes an upper metal member 1121 and a lower metal member 1122, the pump body assembly 170 is fixedly installed on the lower metal member 1122 or the upper metal member 1121, the lock lever assembly 150 is movably installed on the upper metal member 1121, the upper metal member 1121 is fixedly installed on the lower metal member 1122, and the second metal installation base 211 is hinged to the lower metal member 1122.

Of course, in other embodiments, the first metal installation base 112 may also be formed of left and right metal members by splicing.

Referring to FIGS. 5 to 8, as an example of upper and lower parts fitting together, the lower metal member 1122 is substantially a U-shaped structure, the upper metal member 1121 is substantially an inverted U-shaped structure, and the upper metal member 1121 and the lower metal member 1122 are matched with each other and are fixedly connected by means of a metal member (such as a screw 1124).

Referring to FIGS. 5 to 8, in an embodiment, a front cover metal member 1123 is further included. The front cover metal member 1123 is fixedly connected to the first metal member and the second metal member for mounting associated components on the front cover 111. The front cover metal member 1123 may serve as the front cover 111 of the pump main body 100, or may also be made of a different material from the front cover 111 and the two are then assembled together. For example, the front cover 111 is made of plastic.

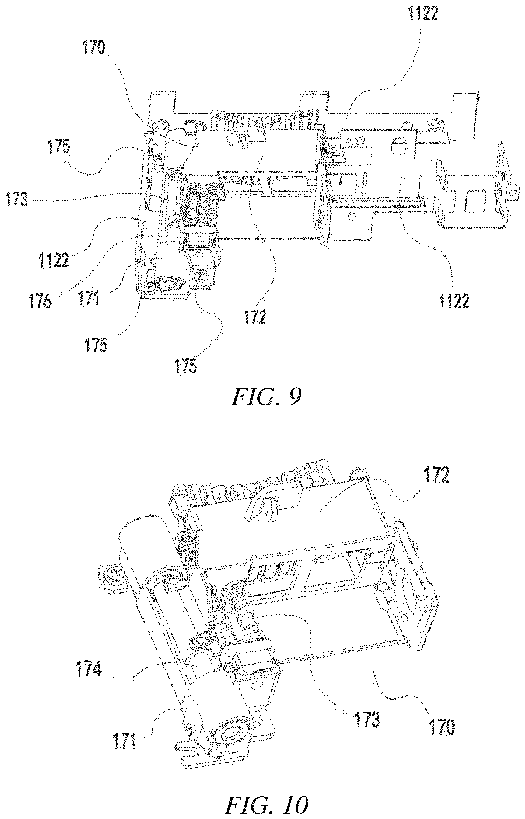

Referring to FIGS. 9 and 10, in an embodiment, a peristaltic pump is used as an example. The pump body assembly 170 includes a pump installation base 171, a peristaltic pump 172, and an elastic member 173, where the pump installation base 171 is fixedly installed on the first metal installation base 112, for example, may be installed on the lower metal member 1122. The peristaltic pump 172 is installed on the pump installation base 171, for example, may be installed on a sliding shaft 176 of the pump installation base 171. The elastic member 173 acts between the peristaltic pump 172 and the first metal installation base 112 and provides an elastic restoring force to the peristaltic pump 172 for driving the peristaltic pump 172 to move towards the tube pressing assembly 220.

In FIGS. 9 and 10, the pump installation base 171 is fixedly installed on the lower metal member 1122 by means of the metal member (such as the screw 175). Of course, it may also be installed on the upper metal member 1121 or other positions of the first metal installation base 112.

Further, the lock lever assembly 150 is movably installed on the first metal installation base 112, such as the upper metal member 1121. During the movement of the lock lever assembly 150, it achieves locking and unlocking between the lock lever assembly and the lock catch mechanism on the pump door 200, so as to lock the pump door 200 to and unlock same from the pump main body 100.

The lock lever driving mechanism 160 typically uses an electric motor, where the electric motor drives a lead screw, the lead screw drives a matching nut, the nut is connected to a lock lever of the lock lever assembly 150, and the lock lever translates along with the nut to achieve locking and unlocking therewith a lock catch on the pump door 200, thereby achieving opening and closing of the pump door 200 of the infusion pump. Due to a large force of the lock lever to open or close the door and a large load on the lock lever, a traditional method is to directly and rigidly fix the nut on the lock lever. When the direction of movement of the lock lever cannot keep parallel to the lead screw, the nut and lead screw cooperate to generate a torque, and as a result, the electric motor needs to output a greater torque to drive the lock lever to move. During actual movement of the lock lever, it is difficult to ensure that the lock lever is parallel to the lead screw, and this rigid connection method will generate a torque on the nut and the lead screw. If a torque margin of the electric motor is insufficient, a stuck or unsmooth door opening and closing will occur; if the torque margin of the electric motor is excessively large, the power consumption of the system will be increased, which also increase the volume of the electric motor and is inconvenient to miniaturize the device.

In this regard, in an embodiment, the lock lever driving mechanism 160 has a driving member and an output member connected to the driving member in a transmission manner. The lock lever assembly 150 is rotatably installed on the output member, and the output member drives the lock lever assembly 150 to move in a straight reciprocating motion along a first axis in a horizontal plane to enable the lock lever assembly 150 to be locked to the lock catch or unlocked from the lock catch. The first axis passes through the axis of the output member, for example as shown in FIGS. 11 and 12, in an embodiment, the output member is a nut 163 of a lead screw and nut transmission mechanism. The first axis passes through the axis of the nut 163, that is, coincides with the central axis of the lead screw 162 mating with the nut 163. Of course, the output member may also be other components, such as a piston rod in a cylinder, a hydraulic cylinder, in which case the first axis coincides with the central axis of the piston rod. The center line of rotation of the lock lever assembly 150 relative to the output member is located in the same horizontal plane as the first axis and perpendicular to the first axis.

The lock lever assembly 150 is rotatably installed on the output member, and the center line of rotation of the lock lever assembly 150 relative to the output member is located in the same horizontal plane as the first axis and perpendicular to the first axis. When the lock lever assembly 150 is inclined to an angle by an external force, the lock lever assembly 150 can rotate relative to the output member so as to automatically adjust an axis fitting structure with the output member in this rotary connection manner, and the output member will not be driven to deflect about the center line of rotation; the counter-acting force of the lock lever assembly 150 against the output member will not generate a moment deviating from the center of the output member, so that the output member can be prevented from deflecting, then the output member will not cause an eccentric moment to a component (such as the lead screw 162 in the following description or a piston cylinder in other embodiments) driving the output member, and finally a series of problems caused by deflection of the output member are avoided.

Further, the output member and the lock lever assembly 150 are in line contact and/or point contact therebetween, contact positions of the line contact and/or contact positions of the point contact are symmetrically distributed on two sides of the first axis and located in the same horizontal plane as the first axis, and a connecting line of the contact positions between the output member and the lock lever assembly 150 is perpendicular to the first axis.

The driving mechanism may be selected from, but not limited to, an electric motor transmission structure, for example, the cylinder or the hydraulic cylinder as shown in the foregoing description, or even an electromagnet or other structures capable of outputting the straight reciprocating motion.

Referring to FIGS. 11 and 12, in an embodiment, the driving mechanism includes a lead screw and nut transmission mechanism, the driving member is the electric motor 161, the nut 163 in the lead screw and nut transmission mechanism serves as the output member, the lock lever assembly 150 is installed on the nut 163, the electric motor 161 drives the lead screw 162 in the lead screw and nut transmission mechanism so as to drive the nut 163 and the lock lever assembly 150 to move, and the first axis coincides with the central axis of the lead screw 162 in the lead screw and nut transmission mechanism.

Further, as an example of a rotatable connection, in an embodiment, at least one of the nut 163 and the lock lever assembly 150 has a protrusion 1631, the other has a bayonet 1521 corresponding to the protrusion 1631, the bayonet 1521 is rotatably sleeved on an outer wall of the protrusion 1631, and an inner wall of the bayonet 1521 is in line contact with the outer wall of the protrusion 1631. In the structure shown in FIGS. 11 and 12, two sides of the nut 163 are respectively provided with the protrusion 1631, and the lock lever assembly 150 is provided with the bayonet 1521 corresponding to the protrusion 1631.

In order to ensure the line contact, in an embodiment, at least one of the inner wall of the bayonet 1521 and the outer wall of the protrusion 1631 has an arc-shaped wall body. Referring to FIGS. 11 and 12, the protrusion 1631 is in a cylindrical shape, and the arc-shaped outer wall thereof is in line contact with the inner wall of the bayonet 1521. Of course, in some other embodiments, only the portion of the protrusion 1631 in contact with the inner wall of the bayonet 1521 may also be configured as an arc-shaped wall body, and other non-contact portions may be adaptively in other shapes. In addition, the inner wall of the bayonet 1521 may also be configured to have an arc-shaped wall body, and the protrusion 1631 may be configured as an arc-shaped wall body, or may be configured in other shapes, such as planar, so as to realize line contact therebetween. In some embodiments, the bayonet 1521 may also be a circular hole, and the protrusion 1631 may be a circular shaft, such that the two both have an arc-shaped wall body. At this time point, the bayonet 1521 is slightly larger than the protrusion 1631 such that the protrusion 1631 can move in the bayonet 1521 to automatically adjust the position on the nut 163 after the lock lever assembly 150 receives force.

In addition to the line contact described above, it is also possible that the output member is in point contact with the lock lever assembly 150. For example, the contact positions on the output member and the lock lever assembly 150 may each be in a spherical shape to form ball-to-ball contact so as to form the point contact. The point contact positions are symmetrically distributed on two sides of the first axis, and the connecting lines of all the contact points are in the same horizontal plane as the first axis and perpendicular to the first axis.

Referring to FIGS. 11 and 12, in an embodiment, the bayonet 1521 has a U-shaped opening, and the U-shaped bayonet 1521 is sleeved on the protrusion 1631.

Referring to FIG. 13, in an embodiment, the lead screw 162 has rectangular gear teeth 1621. The transfer efficiency of the rectangular gear teeth 1621 is higher than that of triangular teeth and trapezoidal teeth, which can improve the efficiency of transmission between the lead screw 162 and the nut 163.

Referring to FIG. 11, in an embodiment, the electric motor 161 is connected to the lead screw 162 of the lead screw and nut transmission mechanism in a transmission manner by means of a gear transmission mechanism 164. In other embodiments, a pulley mechanism, a sprocket wheel mechanism, etc. may also be used for transmission of movement.

Further, the lock lever assembly 150 is snap-fitted with the lock catch assembly 220. Therefore, the lock catch assembly 220 has a lock catch with a hook portion therein, and the corresponding lock lever assembly 150 has a snap-fit portion that fits to the lock catch. Referring to FIGS. 11 and 12, in an embodiment, the lock lever assembly 150 includes a lock lever 151 and a connecting member 152, where the snap-fit portion is disposed on the lock lever 151. The lock lever 151 is connected to the connecting member 152, and the connecting member 152 is rotatably connected to the output member (such as the nut 163), such that the output member drives the connecting member 152 and the lock lever 151 to move. The structure of the lock lever assembly 150 connected to the output member may be specifically disposed on the connecting member 152. The lock lever 151 may be fixedly connected to or integrally formed with the connecting member 152. The fixed connection between the connecting member 152 and the lock lever 151 may be achieved in various manners including, but not limited to, screw fastening, snap-fitting, welding, bonding, etc.

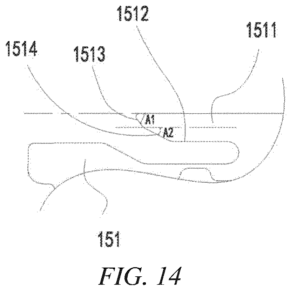

Referring to FIG. 14, in an embodiment, the snap-fit portion has a snap-fit fastener 1511, and the snap-fit fastener 1511 is a hook-like structure. The snap-fit fastener 1511 has a snap-fit surface 1512 configured to be in contact with the corresponding lock catch assembly 220 and to fasten the corresponding lock catch assembly 220. Referring to FIGS. 6 and 8, when the pump door 200 and the pump main body 100 need to be locked, the lock catch assembly 220 is first moved to an entrance of the snap-fit fastener 1511, the lock lever driving mechanism 160 then drives the lock lever 151 to move towards the locking position, and as shown in the view of FIG. 8, the lock lever 151 moves towards the locking position from right to left. The snap-fit surface 1512 is an inclined plane such that the lock catch assembly 220 is driven to gradually approach the side where the lock lever 151 is located, and is finally fastened into a slot of the snap-fit fastener 1511. When unlocking is needed, the lock lever driving mechanism 160 drives the lock lever 151 to move towards the unlocking position, and as shown in the view of FIG. 8, the lock lever 151 moves from left to right so as to release the lock catch assembly 220, and at this time point the pump door 200 can be opened.

Since the resistance to the movement of the pump door 200 towards the pump body during locking mainly comes from the counter-acting force against the lock catch assembly 220 after the infusion tube 300 is pressed, the lock catch assembly 220 needs to overcome the counter-acting force and move a distance to the side where the lock lever 151 is located. If the snap-fit surface 1512 has excessively small inclination, the snap-fit surface 1512 should be set very long such that the lock catch assembly 220 can be pulled in position, which will result in an excessively large volume of the entire infusion pump and is thus not conductive to compact design. If the snap-fit surface 1512 has excessively large inclination, as the snap-fit fastener 1511 pulls the lock catch assembly 220 towards the side where the lock lever 151 is located, the snap-fit fastener 1511 will receive a greater (relative to the case of excessively small inclination) acting force from the lock catch assembly 220. In order to overcome this acting force, the lock lever driving mechanism 160 needs to output a greater driving force, increasing the load on the driving member (such as the electric motor 161), which causes an increase in the cost of the lock lever driving mechanism 160.

For this problem, referring to FIG. 14, in an embodiment of the disclosure, the snap-fit surface 1512 has a first inclined surface 1513 and a second inclined surface 1514, where the first inclined surface 1513 is disposed at an outer end of the snap-fit surface 1512, the second inclined surface 1514 is connected to an inward end of the first inclined surface 1513, and the angle of inclination A1 of the first inclined surface 1513 is greater than the angle of inclination A2 of the second inclined surface 1514.

When it needs to lock the pump door 200 to the pump body, the pump door 200 is first moved to the opening of the snap-fit fastener 1511, and the lock lever 151 is then driven to move towards the locking position. The first inclined surface 1513 of the snap-fit fastener 1511 is first in contact with the lock catch assembly 220, and at this stage, the pump door 200 only slightly presses the infusion tube 300, and the counter-acting force from the infusion tube 300 is not large, so that the angle of inclination A1 of the first inclined surface 1513 can be set to be larger, and the lock catch assembly 220 can be quickly pulled a distance, which does not cause excessive load on the driving member. When the lock catch assembly 220 slides to the second inclined surface 1514 along the first inclined surface 1513, the infusion tube 300 has been pressed to a certain extent at this time point, so that its counter-acting force against the pump door 200 and the lock catch assembly 220 is increased. At this time point, since the inclination of the second inclined surface 1514 is smaller than that of the first inclined surface 1513, there is no excessive load on the driving member when the lock catch assembly 220 is pulled. Finally, after passing through the second inclined surface 1514, the lock catch slides into the groove of the snap-fit fastener 1511 to complete locking.

The inclination of the first inclined surface 1513 is larger such that the lock catch assembly 220 and the pump door 200 can be quickly pulled to a certain position, and then the lock catch assembly 220 can be slowly pulled through the second inclined surface 1514. This combination structure reduces the stroke of the lock catch assembly 220 on the snap-fit surface 1512, therefore, the lock lever 151 does not need to be made too long, which is conducive to the compactness of the device and also reduces the load on the driving member (such as the electric motor 161) to prevent damage to the driving member and also reduce the cost of the lock lever driving mechanism 160.

Referring to FIGS. 11 and 12, in an embodiment, the lock lever 151 is of an elongated sheet structure having at least two snap-fit portions disposed on the side of the lock lever 151 facing the corresponding lock catch. Referring to FIG. 5, there are three snap-fit fasteners 1511 shown in the figure, which are disposed on the lock lever 151 at equal intervals. Three corresponding lock catches are also provided and correspond to the snap-fit fasteners 1511 in position.

The disclosure has been described by using specific examples above, which are merely for the purpose of facilitating understanding of the disclosure and are not intended to limit the disclosure. For those skilled in the art, changes may be made to the specific embodiments described above in accordance with the concept of the disclosure.

## Claims

1. An infusion pump, **characterized in that** the infusion pump comprises a pump main body and a pump door mounted on the pump main body in a lockable and unlockable manner, wherein
the pump main body comprises a pump main body housing, a control module, an external interface assembly, a power supply module, a battery assembly, a lock lever assembly, a lock lever driving mechanism, and a pump body assembly; the pump main body housing has an installation cavity, a side of the pump main body housing facing the pump door is a front cover, and the front cover has an infusion tube installation structure for installing an infusion tube and a pump main body installation position for installing the pump body assembly; the control module, the external interface assembly, the power supply module, the battery assembly, the lock lever assembly, the lock lever driving mechanism and the pump body assembly are all installed in the installation cavity; the battery assembly, the pump body assembly and the lock lever driving mechanism are arranged side-by-side behind the front cover, and the pump body assembly is disposed at the pump main body installation position to press the infusion tube; the battery assembly and the lock lever driving mechanism are respectively disposed on two sides of the pump body assembly; the lock lever assembly is located above the pump body assembly and the lock lever driving mechanism, and the lock lever driving mechanism drives the lock lever assembly to move so as to lock the lock lever assembly to or unlock same from the pump door; the power supply module and the external interface assembly are arranged side-by-side, one of which is located behind the pump body assembly, and the other is located behind the lock lever driving mechanism; and
the pump door has a door body, a lock catch assembly and a tube pressing assembly, the lock catch assembly and the tube pressing assembly are installed on the door body, the lock catch assembly is configured to be locked to the lock lever assembly, and the tube pressing assembly is disposed corresponding to a position of the pump body assembly and is configured to press the infusion tube together with the pump body assembly.

2. The infusion pump of claim 1, the pump main body further comprises a fluid stop assembly, wherein the front cover is divided into an upstream region and a downstream region, with the pump body assembly as a boundary, according to a flow direction of fluid in the infusion tube, and the fluid stop assembly is located in the downstream region of the front cover and exposed from the front cover so as to stop fluid flow in the infusion tube.

3. The infusion pump of claim 2, the fluid stop assembly is installed in a gap between the lock lever driving mechanism and the front cover.

4. The infusion pump of claim 2, the pump main body further comprises an upper pressure measurement assembly and a lower pressure measurement assembly, wherein the upper pressure measurement assembly is located in the upstream region of the front cover, the lower pressure measurement assembly is located in the downstream region of the front cover and located upstream of the fluid stop assembly, and the upper pressure measurement assembly and the lower pressure measurement assembly are exposed from the front cover so as to measure a pressure of fluid in the infusion tube; and the door body is provided with an upper pressure measurement press block corresponding to the upper pressure measurement assembly and a lower pressure measurement press block corresponding to the lower pressure measurement assembly.

5. The infusion pump of claim 4, the upper pressure measurement assembly is installed in a gap between the battery assembly and the front cover.

6. The infusion pump of claim 4, wherein the lower pressure measurement assembly is installed in a gap between the lock lever driving mechanism and the front cover.

7. The infusion pump of claim 4, the pump main body further comprises an upper ultrasound detection assembly and a lower ultrasound detection assembly, wherein the upper ultrasound detection assembly is located in the upstream region of the front cover and located downstream of the upper pressure measurement assembly; the lower ultrasound detection assembly is located in the downstream region of the front cover and located upstream of the lower pressure measurement assembly; the upper ultrasound detection assembly and the lower ultrasound detection assembly are exposed from the front cover so as to detect air bubbles in the infusion tube; and the door body is provided with an upper ultrasound detection press block corresponding to the upper ultrasound detection assembly and a lower ultrasound detection press block corresponding to the lower ultrasound detection assembly.

8. The infusion pump of claim 4, wherein the pump main body further comprises an upper ultrasound detection assembly and a lower ultrasound detection assembly, wherein the upper ultrasound detection assembly is located in the upstream region of the front cover and located upstream of the upper pressure measurement assembly; the lower ultrasound detection assembly is located in the downstream region of the front cover and located between the lower pressure measurement assembly and the fluid stop assembly; the upper ultrasound detection assembly and the lower ultrasound detection assembly are exposed from the front cover so as to detect air bubbles in the infusion tube; and the door body is provided with an upper ultrasound detection press block corresponding to the upper ultrasound detection assembly and a lower ultrasound detection press block corresponding to the lower ultrasound detection assembly.

9. The infusion pump of claim 7 or 8, wherein the upper ultrasound detection assembly is installed in a gap between the battery assembly and the front cover.

10. The infusion pump of claim 7 or 8, wherein the lower ultrasound detection assembly is installed in a gap between the lock lever driving mechanism and the front cover.

11. The infusion pump of claim 7 or 8, wherein the pump main body further comprises an upper tube in-position detection assembly and a lower tube in-position detection assembly, wherein the upper tube in-position detection assembly is located in the upstream region of the front cover and located upstream of the upper pressure measurement assembly and the upper ultrasound detection assembly; the lower tube in-position detection assembly is located in the downstream region of the front cover and located downstream of the fluid stop assembly; and the upper tube in-position detection assembly and the lower tube in-position detection assembly are exposed from the front cover so as to detect whether the infusion tube is installed in position.

12. The infusion pump of claim 11, wherein the upper tube in-position detection assembly is installed in a gap between the battery assembly and the front cover.

13. The infusion pump of claim 11, wherein the lower tube in-position detection assembly is installed on a rear side of the front cover and located on an outer side of the lock lever driving mechanism.

14. The infusion pump of claim 11, wherein the upper tube in-position detection assembly and the lower tube in-position detection assembly are respectively disposed at outermost ends of two sides of the front cover.

15. The infusion pump of claim 11, wherein the pump main body further comprises a drop counting sensor disposed behind the upper tube in-position detection assembly.

16. The infusion pump of any one of claims 1 to 15, wherein at least part of the pump main body housing is a first metal installation base made of a metallic material, and the pump body assembly, the lock lever assembly and the lock lever driving mechanism are installed on the first metal installation base; at least part of the door body of the pump door is a second metal installation base made of a metalic material, and the tube pressing assembly and the lock catch assembly are installed on the second metal installation base;
one end of the first metal installation base is rotatably connected to the second metal installation base to enable the door body to rotate relative to the pump main body; there are a locking position and an unlocking position on a movement path of the second metal installation base; in the locking position, the lock lever assembly is locked to the lock catch assembly, the tube pressing assembly and the pump body assembly are located on two sides of the infusion tube, and when the pump body assembly is started, the infusion tube is pressed so as to perform infusion; and in the unlocking position, the lock lever assembly is unlocked from the lock catch assembly such that the tube pressing assembly can move away from one side of the infusion tube to release the infusion tube.

17. The infusion pump of any one of claims 1 to 15, wherein the pump main body further comprises a first metal installation base, which is installed in the pump main body housing, and on which the pump body assembly, the lock lever assembly, and the lock lever driving mechanism are installed; the pump door comprises a second metal installation base, which is disposed in the door body, and on which the tube pressing assembly and the lock catch assembly are installed;
one end of the first metal installation base is rotatably connected to the second metal installation base to enable the door body to rotate relative to the pump main body; there are a locking position and an unlocking position on a movement path of the second metal installation base; in the locking position, the lock lever assembly is locked to the lock catch assembly, the tube pressing assembly and the pump body assembly are located on two sides of the infusion tube, and when the pump body assembly is started, the infusion tube is pressed so as to perform infusion; and in the unlocking position, the lock lever assembly is unlocked from the lock catch assembly such that the tube pressing assembly can move away from one side of the infusion tube to release the infusion tube.

18. The infusion pump of claim 16 or 17, the first metal installation base comprises an upper metal member and a lower metal member, the pump body assembly is fixedly installed on the lower metal member or the upper metal member, the lock lever assembly is movably installed on the upper metal member, the upper metal member is fixedly installed on the lower metal member, and the second metal installation base is hinged to the lower metal member.

19. The infusion pump of claim 18, the lower metal member is substantially of a U-shaped structure, the upper metal member is substantially of an inverted U-shaped structure, and the upper metal member and the lower metal member are matched with each other and fixedly connected by means of a metal member.

20. The infusion pump of any one of claims 16-19, the lock lever driving mechanism has a driving member and an output member connected to the driving member in a transmission manner, the lock lever assembly is rotatably installed on the output member, and the output member drives the lock lever assembly to move in a straight reciprocating motion along a first axis in a horizontal plane to enable the lock lever assembly to be locked to the lock catch or unlocked from the lock catch; the first axis passes through an axial center of the output member; and the lock lever assembly is located, relative to a center line of rotation of the output member, in the same horizontal plane as the first axis and perpendicular to the first axis.

21. The infusion pump of claim 20, the output member and the lock lever assembly are in line contact and/or point contact therebetween, contact positions of the line contact and/or contact positions of the point contact are symmetrically distributed on two sides of the first axis and located in the same horizontal plane as the first axis, and a connecting line of the contact positions between the output member and the lock lever assembly is perpendicular to the first axis.

22. The infusion pump of claim 21, the lock lever driving mechanism comprises a lead screw and nut transmission mechanism, wherein an electric motor serves as the driving member, a nut in the lead screw and nut transmission mechanism serves as the output member, the lock lever assembly is installed on the nut, the electric motor drives a lead screw in the lead screw and nut transmission mechanism so as to drive the nut and the lock lever assembly to move, and the first axis coincides with a central axis of the lead screw in the lead screw and nut transmission mechanism.

23. The infusion pump of claim 22, two sides of the nut are respectively provided with a protrusion, the lock lever assembly is provided with a bayonet corresponding to the protrusion, the bayonet is sleeved on an outer wall of the protrusion, and an inner wall of the bayonet is in line contact with the outer wall of the protrusion.

24. The infusion pump of claim 23,the bayonet has a U-shaped opening fastened and sleeved on the protrusion.

25. The infusion pump of claim 20,the lock lever assembly has a snap-fit fastener, which has a snap-fit surface configured to be in contact with the corresponding lock catch assembly and to fasten the snap-fit surface of the lock catch assembly, the snap-fit surface has a first inclined surface and a second inclined surface, the first inclined surface is disposed at an outer end of the snap-fit surface, the second inclined surface is connected to an inward end of the first inclined surface, and an angle of inclination of the first inclined surface is greater than that of the second inclined surface.

26. The infusion pump of claim 25,the lock lever assembly comprises a lock lever and a connecting member, wherein the lock lever has the snap-fit fastener, the lock lever is connected to the connecting member, and the connecting member is rotatably connected to the output member to enable the output member to drive the connecting member and the lock lever to move.

27. The infusion pump of any one of claims 16-26,the pump body assembly comprises a pump installation base, a peristaltic pump and an elastic member, wherein the pump installation base is fixedly installed on the first metal installation base, the peristaltic pump is installed on the pump installation base, and the elastic member acts between the peristaltic pump and the first metal installation base to apply an elastic restoring force to the peristaltic pump for driving the peristaltic pump to move towards the tube pressing assembly.
